# EUROPÄISCHE PATENTSCHRIFT

(11) **EP 1 299 345 B1**
(45) Veröffentlichungstag und Bekanntmachung des Hinweises auf die Patenterteilung: **30.08.2006**
(21) Anmeldenummer: 01936421.5
(22) Anmeldetag: 01.06.2001
(51) Int. Cl.: C07C 213/06, C07C 219/08

(54) **VEFAHREN ZUR HERSTELLUNG VON AMINOALKYL(METH)ACRYLATEN**
METHOD FOR PREPARING AMINO ALKYL(METH)ACRYLATES
PROCEDE DE PRODUCTION D'AMINOALKYL(METH)ACRYLATES

(30) Priorität: 21.06.2000 DE 10029517
(43) Veröffentlichungstag der Anmeldung: 09.04.2003
(73) Patentinhaber: Stockhausen GmbH, 47805 Krefeld (DE)
(72) Erfinder: HOUBEN, Jochen, 47906 Kempen (DE); EICKWINKEL, Ralph, 41334 Nettetal (DE); HOPPE, Olivier, 47807 Krefeld (DE); KUBIAK, Bernd, 47799 Krefeld (DE); KÜSTER, Erich, 47803 Krefeld (DE)
(74) Vertreter: Bülle, Jan
(86) Internationale Anmeldenummer: PCT/EP2001/006237
(87) Internationale Veröffentlichungsnummer: WO 2001/098254

(56) Entgegenhaltungen:
- EP-A- 0 906 902
- EP-A- 0 960 877
- DD-A- 152 540
- DE-A- 2 725 255
- DE-A- 2 752 109
- DATABASE WPI Section Ch, Week 197819 Derwent Publications Ltd., London, GB; Class E16, AN 1978-34311A XP002176946 & JP 53 034714 A (MITSUBISHI CHEM IND LTD) , 31. März 1978 (1978-03-31)
- JUNZO OTERA: "Transesterification" CHEM. REV., Bd. 93, 1993, Seiten 1449-70, XP002176945 in der Anmeldung erwähnt

## Beschreibung

Die Erfindung betrifft ein verbessertes Verfahren zur Herstellung von Aminoalkyl(meth)acrylaten unter Verwendung von Distannoxanen als Katalysator.

Aminoalkyl(meth)acrylate wie z.B. Dimethylaminoethylacrylat und Dimethylaminoethylmethacrylat sind als Ausgangstoffe für kationische Polymere, wie sie z.B. in Flockungsmitteln eingesetzt werden, von großem Interesse. Dabei sind sowohl Homopolymerisate wie auch Copolymerisate mit anderen Monomeren gebräuchlich. Das Dimethylaminoethylacrylat bzw. das Dimethylaminoethylmethacrylat kann direkt oder nach Quaternierung der tertiären Aminofunktion in die Polymerisation eingesetzt werden. Eine solche Quaternierung wird großtechnisch üblicherweise z. B. mit Dimethylsulfat oder Methylchlorid durchgeführt.

Die Herstellung von Dimethylaminoalkyl(meth)acrylat kann wegen des basischen Charakters des Dimethylaminoalkohols nicht durch direkte Veresterung erfolgen, sondern erfordert eine Umesterung des Dimethylaminoalkohols mit den (Meth)acrylsäureestern kurzkettiger Alkohole.

Vor allem bei der Herstellung der Acrylate, aber auch in geringerem Umfang bei der Herstellung der Methacrylate treten bei der Umesterung Nebenreaktionen auf. Diese erklären sich zum überwiegenden Teil aus der Michaeladdition des freigesetzten Alkohols oder des Dimethylaminoalkohols an das gebildete Dimethylaminoalkyl(meth)acrylat bzw. an das als Rohstoff eingesetzte Alkyl(meth)acrylat. Während man die Addition des freigesetzten Alkohols durch geeignete verfahrenstechnische Maßnahmen, d.h. durch schnelle Entfernung des gebildeten Alkohols unterdrücken kann, ist dies beim Dimethylaminoethanol nicht der Fall. Das Verhältnis der Nebenprodukte zum Wertprodukt lässt sich außer über die Reaktionsbedingungen, d.h. vor allem die Reaktionstemperatur auch in großem Maße über die Art des Katalysators steuern.

Aus wirtschaftlichen Gründen muss ein Katalysator selektiv, hochwirksam und wiedereinsetzbar sein. Außerdem sollte ein Katalysator aus Gründen der besseren Dosierbarkeit entweder flüssig oder gut im Aminoalkohol löslich sein. Eine Löslichkeit in dem als Umesterungskomponente gebräuchlichen Ethylacrylat ist nicht ausreichend, da dieser Einsatzstoff einen Flammpunkt unterhalb der Raumtemperatur hat und im Verdacht der Erzeugung bleibender Gesundheitsschäden steht. Aus Sicherheits- und Kostengründen ist es deshalb vorteilhaft, wenn die Katalysatorlösung unterhalb des Flammpunktes z.B. des Dimethylaminoethanols (39°C) hergestellt und gelagert werden kann, ohne dass es zu Feststoffausfällungen kommt. Da aus Gründen der Wirtschaftlichkeit ein Wiedereinsatz des freigesetzten Azeotrops unabdingbar ist, muss von einem Katalysator weiter verlangt werden, dass er während des bestimmungsgemäßen Einsatzes keine Substanzen freisetzt, die sich im Siedebereich des Azeotropes anreichern.

Die üblicherweise zur Umesterung eingesetzten alkalischen Katalysatoren wie Natriummethanolat führen hier zu einer großen Menge an Michaeladdukt, so dass der erhaltene Reaktoraustrag nicht mehr wirtschaftlich zum Zielprodukt aufgearbeitet werden kann.

In der JP 90-405854 und JP 89-256222 wird die Verwendung von Dibutylzinnoxid zur Katalyse der Umsetzung von Dimethylaminoethanol mit Methylacrylat empfohlen. Dieser Katalysator hat den Vorteil einer sehr hohen Reaktivität, führt allerdings zu einer erhöhten Menge von Nebenprodukten und hat zudem den Nachteil, dass er selbst in kleinen Mengen nicht im Dimethylaminoethanol oder anderen Aminoalkoholen löslich ist. Dadurch ist seine technische Verwendung auf Batchherstellverfahren begrenzt, die die chargenweise Zugabe von Feststoffen zum Ansatz erlauben. Die vorstehend genannten technischen Nachteile gelten, zum mindestens teilweise, nicht für das in der DE-OS-27 52 109 beschriebene Verfahren zur Herstellung von Dimethylaminoethylmethacrylat, in dem Di-n-octylzinnoxid als Katalysator verwendet wird bzw. für die gleichfalls zur Herstellung von Dimethylaminoethylmethacrylat in der DE- OS-27 25 255 als Katalysatoren beschriebenen Organozinnverbindungen, wie z.B. Tetrabutylzinn, Trioctylzinnethoxid, Dibutylzinndimethoxid, Dibutylzinndihydrid, Dibutylzinndilaurat, Dibutylzinnmaleat, Bis-(tributylzinn)oxid und Bis-(dibutylmethoxyzinn)oxid, wobei unter Verwendung der vorgenannten Katalysatoren die Umesterung des Methylmethacrylat mittels Dimethylaminoethanol erfolgt sowie die zur Herstellung von Dialkylamiuoalk-ylacrylaten und -methacrylaten in der JP-A-53034714 als Katalysator verwendeten Di-n-Butylzirw.dicarboxylate.

In mehreren Patenten (JP 90-174549; JP 89-317700, JP 88-208221, JP 88-164874, JP 88- , 125971) wird empiohlen, Acetylacetonate verschiedener Übergangsmetalle als Katalysatoren einzusetzen. Obwohl diese Katalysatoren sehr reaktiv sind, haben sie den Nachteil, in der Reaktionslösung Acetylaceton abzuspalten, welches sich nachteiligerweise im Azeotrop wiederfindet und dessen aus wirtschaftlichen Gründen notwendige Verwendung zur Herstellung von frischem Alkyl(meth)acrylat unmöglich macht.

Des weiteren verlieren diese Katalysatoren beim Einsatz sehr schnell ihre Wirksamkeit, was einen Wiedereinsatz ausschließt und teilweise sogar eine Nachdosierung verlangt, um die Reaktion bis zu einem akzeptablen Umsatz zu fuhren.

Das in der DE 38 74 827 beschriebene Orthoethyltitanat katalysiert die Umesterung mit Ethylacrylat sehr selektiv, steht aber in der Reaktionsgeschwindigkeit deutlich hinter dem Dibutylzinnoxid zurück. Deshalb müssen von diesem Katalysator entweder größere Mengen eingesetzt oder aber wesentlich längere Reaktionszeiten in Kauf genommen werden oder mit relativ großen Überschüssen an Alkylacrylat gearbeitet werden. Beides ist aus wirtschaftlichen Gesichtspunkten her ungünstig.

Nachteilig an den Titanaten ist zudem ihre leichte Hydrolysierbarkeit, da durch die Ausgangsstoffe, speziell dem hygroskopischen Aminoalkohol stets Spuren von Wasser ins System eingeschleust werden. So enthält das technisch verfügbare Dimethylaminoethanol Wasser in der Größenordnung von ca. 500 ppm. Diese Wasserspuren führen dazu, dass sich festes Titandioxid bildet, welches beim Handling des Reaktoraustrages oder des Destillationssumpfes zu erheblichem Verschleiß an Rohrleitungen und Pumpen führt. Durch diese Hydrolyse ist ein aus ökonomischen und ökologischen Gründen anzustrebender zumindest teilweiser Wiedereinsatz des Destillationssumpfes nur nach aufwendiger Reinigung (Filtration) möglich. Ferner kann das Tetraethyltitanat nur bei der Umesterung mit Ethylacrylat Verwendung finden, da es in der Reaktionslösung relativ schnell Ethanol abspaltet, welches in das zu recyclierende Destillat gelangt.

In der EP 118 639 und EP 160 427 wird ein verbesserter Katalysator auf Titanatbasis beschrieben, der durch Umesterung von Tetraethyltitanat mit Dimethylaminoethanol hergestellt wird. Dieser so hergestellte Katalysator kann unabhängig von der Art des verwendeten Alkyl(meth)acrylats eingesetzt werden, hat aber genau wie das Tetraethyltitanat die Nachteile der leichten Hydrolysierbarkeit und der geringen Reaktivität, was wiederum zu einer schlechten Wiedereinsetzbarkeit des Sumpfes führt.

In der JP 90-100727 wurde ein Verfahren vorgeschlagen, Dimethylaminoethylacrylat aus Dimethylaminoethanol und Methylacrylat unter Einsatz von Lipase M als Katalysator herzustellen. Dieses Verfahren konnte sich aber nicht durchsetzen.

Der Erfindung liegt daher die Aufgabe zugrunde, ein Verfahren zur Herstellung von Aminoalkyl(meth)acrylaten zur Verfügung zu stellen, das unter Verwendung eines oder mehrer Katalysatoren, die die oben genannten Nachteile des Standes der Technik nicht aufweisen, insbesondere im großtechnischen Prozess eine wirtschaftliche Arbeitsweise ermöglicht.

Die Aufgabe wird erfindungsgemäß gelöst durch Umesterung von C1-C4-Alkyl(meth)acrylaten mit Aminoalkoholen, wobei die Umesterung in Gegenwart von mindestens einem Distannoxan der allgemeinen Formel (1) 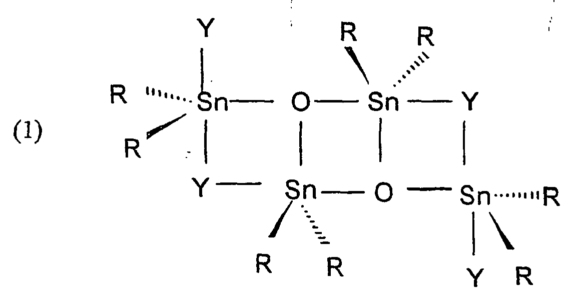 R = linearer, cyclischer oder verzweigter Alkylrest mit 1 bis 6 Kohlenstoffatomen, Phenylrest, wobei R identisch oder verschieden voneinander sein kann,
Y = Halogen, vorzugsweise Cl, Br; Pseudohalogen, vorzugsweise SCN; OH, OAc oder OR, wobei Y identisch oder verschieden voneinander sein kann, als Katalysator durchgeführt wird.

Die katalytische Wirkungsweise der Stoffldasse der Distannoxane wurde bereits in mehreren Artikeln beschrieben. So wurden beispielsweise unter Verwendung von Distannoxanen mit einem hohen Überschuß an Ethylenglykol aus Aldehyden in hohen Ausbeuten Acetale erhalten (Tetrahedron 48, 1992, 1449-1456). Es wurde weiter gefunden, dass die Selektivität der Distannoxane bei der Verseifung von Acetaten bei steigendem Abstand der Funktionalitäten stark abnimmt (J.Chem.Soc.,Chem.Commun, 1991, 1742-1743). Es konnte ebenfalls gezeigt werden, dass Distannoxane auch bei der Umesterung von Carbonsäureester niedrigsiedender Alkohole mit hochsiedenden Alkoholen eingesetzt werden können (J.Org.Chem. 1991, 56, 5307-5311). In diesen Arbeiten wurden weder der Einsatz einer Carbonsäure bzw. eines Carbonsäureesters mit einer der Michaeladdition zugänglichen Doppelbindung noch die Verwendung von Aminoalkohol als Umesterungskomponente beschrieben.

Es war daher überraschend, dass Distannoxane der allgemeinen Formel (1) als Katalysatoren für die Herstellung von Aminoalkyl(meth)acrylaten, insbesondere auch in einem technischen Großprozess geeignet sind bzw. wirtschaftlich eingesetzt werden können, da bereits von Pereyre et al. (Bull.Soc.Chim.Fr. 1969,262-263) hinsichtlich Tributylzinnalkoxiden, die den Distannoxanen chemisch eng verwandt sind, beschrieben worden ist, dass bei der Umesterung von Acrylsäureester nur unakzeptable Ausbeuten zwischen 30 und 70% erhalten worden sind.

Die Herstellung der erfindungsgemäßen Distannoxane der allgemeinen Formel (1), die u.a. einfach durch Umsetzung von Organozinnoxidverbindungen mit den entsprechenden Organozinnhalogenidverbindungen erhältlich sind, ist in der Literatur beschrieben (Chem.Rev. 1993, 93, 1449-1470 und hierin zitierte Literatur).

Bevorzugte Distannoxane sind Diisopropyldibutyltetrachlordistannoxan, Düsopropyldimethyltetrachlordistannoxan, Dibutyldioctyltetrachlordistannoxan, Dimethyldioctyltetrachlordistannoxan und Dimethyldibutyltetrachlordistannoxan, die vorzugsweise allein, aber auch als Katalysatormischung im Verfahren eingesetzt werden können. Die Einsatzmenge des Katalysators beträgt 0,1 bis 10 mmol, vorzugsweise 0,2 bis 1 mmol pro Mol Aminoalkohol. Vorzugsweise wird das Octabutyltetrachlordistannoxan (R = n-Butyl, Y= Cl) als Katalysator eingesetzt.

Als erfindungsgemäße Aminoalkohole kommen alle tertiären Amine in Frage, die mindestens eine primäre Alkoholfunktion aufweisen, wie Dialkylmonoalkanolamine, Monoalkyldialkanolamine oder Trialkanolamine, wobei Alkyl für einen linearen oder verzweigten Alkylrest mit C₁-C₂₂-Kohlenstoffatomen und Alkanol für Ethanol oder Propanol stehen kann. Aus der Gruppe der Dialkylmonoalkanolamine können hier vorzugsweise, aber nicht abschließend genannt werden:
Dimethylaminoethanol, Dimethylaminopropanol, Diethylaminoethanol, Diethylaminopropanol, 3-(Dimethylamino)-2,2-dimethyl-1-propanol.
Beispielhaft, aber nicht ausschließlich sind aus der Gruppe der Monoalkyldialkanolamine N-Methyldiethanolamin oder C₁₂C₁₄-diethanolamin und als Trialkanolamine das Triethanolamin zu nennen.
Erfindungsgemäß werden als C1-C4-Alkyl(meth)acrylate beispielsweise aber nicht ausschließlich Methylacrylat, Methylmethacrylat, Ethylacrylat, Isopropylacrylat oder n-Butylacrylat, eingesetzt, wobei Ethylacrylat bevorzugt ist.

Beim Herstellungsverfahren wird der Alkylester mit Ausnahme des Isopropylacrylatesters vorzugsweise im stöchiometrischen Überschuß eingesetzt werden, da ein gewisser Anteil mit dem entstehenden Alkohol azeotrop im Destillat ausgetragen wird. Der einzusetzende stöchiometrische Überschuß ist hierzu in Abhängigkeit vom Alkylrest je nach Lage des Azeotropes unterschiedlich zu wählen, was dazu führt, dass z.B. beim Methylacrylat ein größerer Überschuß einzusetzen ist, als beim Ethylacrylat. Die Lage der theoretisch zu erzielenden Azeotrope kann experimentell ermittelt oder einschlägigen Standardwerken (z.B. Advances in Chemistry Series 116, Azeotropic Data-III) entnommen werden. In der technischen Auslegung wird man aus wirtschaftlichen Gründen z.B. beim Ethylacrylat oft darauf verzichten, das theoretisch bei Normaldruck zu erzielende Azeotrop mit 72,7% Ethanol abzudestillieren, sondern unter Kostenoptimierung der theoretischen Bodenzahl und des Rücklaufverhältnisses ein Gemisch mit 65-70% Ethanol abziehen.

Die Reaktion der Umesterung kann schon bei niedrigen Temperaturen wie z.B. Raumtemperatur ablaufen, führt aber ohne die destillative Entfernung des entstehenden kurzkettigen Alkohols zu einem Gleichgewicht, welches überwiegend auf der Seite der Edukte liegt. Ebenfalls werden bei niedrigen Temperaturen relativ lange Zeiten bis zur Erreichung des Gleichgewichts benötigt und/oder relativ große und damit unwirtschaftliche Mengen an Katalysator. Auf der anderen Seite wirken sich niedrige Reaktionstemperaturen bei sonst gleichen Rahmenbedingungen positiv auf die Selektivität der Reaktion aus. Die Reaktion wird deshalb bevorzugt zwischen 60 und 180°C durchgeführt, wobei die Umsetzung bei einem Druck im Bereich von 100 bis 1100 mbar, vorzugsweise bei einem Druck im Bereich von 400 bis 1100 mbar, stattfindet.

Als erfindungsgemäße Polymerisationsinhibitoren werden übliche Inhibitoren verwendet, wie z.B. Phenothiazin, tert.-Butylcatechol, Hydrochinonmonomethylether, Hydrochinon, Methylenblau, Kupfersulfat, Eisensulfat, allein oder als Inhibitorenmischung in einer Einsatzmenge von 250 bis 5000 ppm, vorzugsweise 250 bis 2500 ppm, wobei eine Einsatzmenge von 1000 ppm bezogen auf die Gesamtcharge besonders bevorzugt ist.

Die Umsetzung kann im technischen Maßstab batchweise, semibatchweise oder kontinuierlich durchgeführt werden. Aus wirtschaftlichen Gründen wird man bei angestrebten höheren Jahresproduktionen an einem Alkylamino(meth)acrylat stets versuchen, dass Verfahren vollkontinuierlich zu gestalten.

Bei der batchweisen Durchführung der Umsetzung und Aufarbeitung hat man den Nachteil, dass die gebildeten Hochsieder, bei denen es sich vor allem um Michaeladditionsprodukte handelt, unter der längeren thermischen Belastung wieder zerfallen und niedrigsiedende Edukte freisetzen, die anschließend das Wertprodukt verunreinigen. Von den niedrigsiedenden Verunreinigungen sind vor allem die als Edukte eingesetzten leicht flüchtigen (Meth)acrylsäureester von Nachteil, da diese bei der nachfolgenden Alkylierung nicht umgesetzt werden und so durch ihren Geruch und aufgrund ihrer negativen toxikologischen Eigenschaften sowie ihres niedrigen Flammpunktes das von der wässrigen Monomerlösung ausgehende Gefährdungspotential gewaltig erhöhen. Die Bildung der niedrigsiedenden Spaltprodukte kann man vermeiden, indem man nach der Umsetzung zunächst den Reaktoraustrag in einer schonenden Destillation - z.B. mittels Fallfilm- oder Dünnschichtverdampfer - wobei die Verweilzeit mit thermischer Belastung kleiner als 10 Minuten ist, von den Hochsiedern abtrennt und erst danach der fraktionierenden Destillation zur Reinigung des Wertproduktes unterwirft.

In einer bevorzugten Ausführungsform des Verfahrens werden bei der vorgenannten kontinuierlichen Aufarbeitung zunächst die Hochsieder in einem Destillationsschritt abgetrennt, dessen Verweilzeit mit thermischer Belastung kleiner als 5 Minuten, besonders bevorzugt kleiner als 1 Minute ist.

Bei der kontinuierlichen Ausgestaltung der Reaktion ist es sowohl möglich, die Reaktion in einem Durchflussreaktor mit aufgesetzter Kolonne zu betreiben oder vorteilhafterweise diesem ersten Reaktor einen oder mehrere ähnlich konstruierte Reaktoren nachzuschalten, um die Reaktion zu höheren Umsätzen zu bringen oder im optimalen Fall sogar zu einem vollständigen Umsatz. Dabei kann es technisch sinnvoll sein, diese Reaktoren auf demselben Temperaturniveau aber auf unterschiedlichen Druckniveaus zu betreiben, wobei der Druck von Reaktor zu Reaktor vermindert wird. Ebenfalls ist die Auslegung der Reaktion in einer Reaktivkolonne möglich, wobei das Eduktgemisch in der Mitte eingespeist wird und der gebildete leichtsiedende Alkohol die Kolonne über Kopf des Auftriebsteils als Azeotrop verlässt, während das umgesetzte Reaktionsgemisch mit dem Wertprodukt am Fuß des Abtriebsteils entnommen wird. Der geschulte Anlagenbauer kann es vorsehen, zwischen den kontinuierlich betriebenen Reaktionsteil und der ebenfalls kontinuierlich betriebenen Aufarbeitung einen Puffertank mit geeigneten Abrriaßen zu setzen.

In einer bevorzugten Ausgestaltung des Verfahrens werden die hergestellten Aminoalkyl(meth)acrylate in einem kontinuierlichen Destillationsprozeß aufgearbeitet, und die anfallende Leichtsiederfraktion wird in die Reaktion zurückgeführt.

In einer weiteren Ausführungsform wird die anfallende Sumpffraktion zu 1 bis 100%, vorzugsweise zu 25 bis 95% besonders bevorzugt zu 50 bis 90% in die Reaktion zurückgeführt.

Die nach dem erfindungsgemäßen Verfahren hergestellten Aminoalkyl(meth)acrylate, insbesondere Dimethylaminoethylacrylat und Dimethylaminoethylmethacrylat werden zur Herstellung von kationischen Monomeren verwendet.

Die im erfindungsgemäßen Verfahren als Katalysatoren verwendeten Distannoxane der allgemeinen Formel (1) weisen keinen der im Stand der Technik bekannten anderen Katalysatoren anhaftenden Nachteile auf. Insbesondere weisen sie eine höhere Selektivität als die bislang eingesetzten Katalysatoren und eine gleich große Reaktivität wie Dibutylzinnoxid auf. Da sie ihre Aktivität auch in Gegenwart prozessüblicher Wassermengen nicht verlieren, lässt sich der Destillationssumpf im Gegensatz zu den Umsetzungen unter Titanatkatalyse ohne Vorbehandlung recyclieren. Ein weiterer Vorteil ist ihre geringe Giftigkeit. Insbesondere das Octabutyltetrachlordistannoxan ist schon bei Raumtemperatur im Gegensatz zum entsprechenden Dibutylzinnoxid in Konzentrationen von über 10% im Alkylaminoethanol löslich, was eine problemlose Dosierung in den Reaktor ermöglicht.

### Experimentelles:

### Beispiel 1:

### Herstellung des Katalysators Octabutyltetrachlordistannoxan

In einem Rührreaktor werden 250 g n-Heptan vorgelegt und 24,89 g Dibutylzinnoxid und 30,80 g Dibutylzinndichlorid zugegeben. Der Ansatz rührt 16 Stunden lang bei Raumtemperatur (RT), woraufhin sich der Anfangs vorhandene Feststoff (Dibutylzinnoxid) fast vollständig aufgelöst hat. Es wird von wenig noch vorhandenem Feststoff abfiltriert und bis auf 100 ml Volumen eingeengt. Der beim Abkühlen ausgefallene Feststoff wird abgenutscht und im Umlufttrockenschrank bei 70°C zwei Stunden lang getrocknet. Ausbeute 54g = 97% der Theorie.

### Beispiel 2:

### Löslichkeit des Katalysators

Eine Mischung aus 12 Gew.-% des erfindungsgemäßen Katalysators gemäß Beispiel 1 und 88 Gew.-% Dimethylaminoethanol wurde vermischt und 1 h bei 38°C Rühren gelassen. Es resultierte eine klare, feststofffreie Lösung.

### Vergleichsbeispiel 1:

### Schlechte Löslichkeit des Dibutylzinnoxids

Eine Mischung aus 5 Gew.-% Dibutylzinnoxid und 95 Gew.-% Dimethylaminoethanol wurde zunächst 3 h bei RT und anschließend 3 h bei 39°C gerührt. Das Dibutylzinnoxid löste sich nicht in Dimethylaminoethanol.
Eine Mischung aus 2,5 Gew.-% Dibutylzinnoxid und 97,5 Gew.-% Dimethylaminoethanol wurde zunächst 3 h bei RT und anschließend 3 h bei 39°C gerührt. Das Dibutylzinnoxid löste sich nicht in Dimethylaminoethanol.
Die gleichen Versuche wurden 10%-ig in Ethanol und 10%-ig in Ethylacrylat durchgeführt. Es konnte in keinem Fall eine Lösung erhalten werden.

### Beispiel 3:

### Herstellung des Katalysators Diisopropyldibutyltetrachlordistannoxan

1,04 g Diisopropylzinnoxid und 1,43 g Dibutylzinndichlorid werden in 50 ml Toluol zwei Tage im 100 ml Rundkolben gerührt. Nachdem sich der Ansatz in eine klare Lösung verwandelt hat, werden 30,1 g Toluol über eine Destillationsbrücke abdestilliert. Der ausgefallene Feststoff wird auf einem Büchnertrichter abfiltriert und anschließend in eine Petrischale fein verteilt und in einem üblichen Laborabzug 72 Stunden lang getrocknet.

### Beispiel 4:

### Herstellung des Katalysators Diisopropyldimethyltetrachlordistannoxan

1,24 g Diisopropylzinnoxid und 1,23 g Dimethylzinndichlorid werden in 50 ml Toluol zwei Tage im 100 ml Rundkolben gerührt. Nachdem sich der Ansatz in eine klare Lösung verwandelt hat, werden 27,5 g Toluol über eine Destillationsbrücke abdestilliert. Der ausgefallene Feststoff wird auf einem Büchnertrichter abfiltriert und anschließend in eine Petrischale fein verteilt und in einem üblichen Laborabzug 72 Stunden lang getrocknet.

### Beispiel 5:

### Herstellung von Dibutyldioctyltetrachlordistannoxan

Dibutyldioctyltetrachlordistannoxan wird analog Beispiel 4 aus Dibutylzinndichlorid und Dioctylzinnoxid in n-Heptan hergestellt.

### Beispiel 6:

### Herstellung von Dimethyldioctyltetrachlordistannoxan

Dimethyldioctyltetrachlordistannoxan wird analog Beispiel 4 aus Dimethylzinndichlorid und Dioctylzinnoxid in n-Heptan hergestellt.

### Beispiel 7:

### Herstellung von Dimethyldibutyltetrachlordistannoxan

Dimethyldibutyltetrachlordistannoxan wird analog Beispiel 4 aus Dimethylzinndichlorid und Dibutylzinnoxid in n-Heptan hergestellt.

### Umesterungen:

Die Synthesen wurden in einem 1-1-Glaskolben mit schnell drehendem Glasrührer D/D = 1/3 durchgeführt. Die Beheizung erfolgte über ein geregeltes Ölbad. Die Brüden wurden über eine mit Edelstahldrahtnetzen (Durchmesser 6 mm) der Firma MSM gefüllten Füllkörperkolonne mit sechs theoretischen Böden (ermittelt mit Testgemisch n-Hexan/n-Heptan) auf einen geregelten Kolonnenkopf geleitet, bei welchem der als Reaktionskoppelprodukt entstehende Alkohol in seinem jeweiligen Azeotrop entnommen wurde. Entsprechend der eingesetzten (Meth)acrylsäureester wurde wahlweise mit oder ohne Vakuum und je nach Trennaufgabe mit unterschiedlichen Rücklaufverhältnissen gearbeitet. Das Destillat und das Sumpfprodukt wurden gaschromatographisch analysiert.

### Beispiel 8:

193,6 g (2,17 mol) Dimethylaminoethanol, 206,4 g (1,61 mol) Butylacrylat, 0,5 g Phenothiazin und 1,25 g des nach Beispiel 1 hergestellten Katalysators werden zusammen vorgelegt und auf eine Sumpftemperatur von 125°C aufgeheizt. Der innere Druck der Anlage wurde im Laufe der Reaktion von 700 auf 400 mbar heruntergenommen. Das Entnahmeverhältnis am Kopf der Kolonne wurde auf 1: 10 festgesetzt. Im Laufe von 15 Stunden wurde 128,1g Destillat entnommen. Der Sumpf hatte folgende Zusammensetzung:

| | |
|---|---|
| n-Butanol | 0,82 Gew.-% |
| Dimethylaminoethanol | 16,75 Gew.-% |
| n-Butylacrylat | 7,85 Gew.-% |
| Dimethylaminoethylacrylat | 68,76 Gew.-% |
| Höhersiedende Verunreinigungen | 5,82 Gew.-% |
| Selektivität | 91,5 % |

### Beispiel 9:

203,2 g (2,28 mol) Dimethylaminoethanol, 296,8 g (2,97 mol) Ethylacrylat, 0,5 g Phenothiazin und 1,25 g des nach Beispiel 1 hergestellten Katalysators wurden zusammen bei Normaldruck vorgelegt und innerhalb sieben Stunden auf eine Sumpftemperatur von 137°C aufgeheizt. Dabei gingen bei einem Rücklaufverhältnis von 1:7 und einer Kopftemperatur von 77,8-78°C 97,3 g Destillat über. Der Sumpf hatte folgende Zusammensetzung:

| | |
|---|---|
| Ethanol | 0,51 Gew.-% |
| Ethylacrylat | 14,27 Gew.-% |
| Dimethylaminoethanol | 7,71 Gew.-% |
| Dimethylaminoethylacrylat | 76,62 Gew.-% |
| Höhersiedende Verunreinigungen | 0,89 Gew.-% |
| Selektivität | 98,83% |

### Vergleichsbeispiel 2:

Es wurde wie im Beispiel 9 verfahren, nur wurde statt des erfindungsgemäßen Katalysators die gleiche Menge Dibutylzinnoxid eingesetzt. Der Sumpf hatte folgende Zusammensetzung:

| | |
|---|---|
| Ethanol | 0,15 Gew.% |
| Ethylacrylat | 4,19 Gew.% |
| Dimethylaminoethanol | 6,90 Gew.-% |
| Dimethylaminoethylacrylat | 84,79 Gew.-% |
| Höhersiedende Verunreinigungen | 3,72 Gew.-% |
| Selektivität | 95,6% |

Es ist ersichtlich, dass der Anteil an höhersiedenden Verunreinigungen deutlich höher als im erfindungsgemäßen Beispiel 9 ist.

### Beispiel 10:

### Wiedereinsatz vom Destillationssumpf und Destillat

Das Produkt aus Beispiel 9 wurde bei 100 mbar über eine Füllkörperkolonne mit sechs theoretischen Böden aufdestilliert. Das bis zu einer Kopftemperatur von 90°C (Rücklaufverhältnis 1:5) anfallende Destillat 2 (6,3% Ethanol; 57,5% Ethylacrylat; 28,6% Dimethylaminoethanol (DMAE); 7,6% Dimethylaminoethylacrylat) wurde für den Wiedereinsatz aufbewahrt. Der nach der Abdestillation des Wertproduktes bis 102°C Kopftemperatur (Rücklauf 4:2) anfallende Sumpf wurde ebenfalls für den Wiedereinsatz aufbewahrt. In vier aufeinanderfolgenden Ansätzen wurde jeweils ¾ des erhaltenen Destillationssumpfes und das ganze erhaltene Destillat 2 wiedereingesetzt. Die im Destillat 2 enthaltene Menge an Ethylacrylat und DMAE wurde beim Ansatz voll berücksichtigt und die Menge an frisch eingesetztem Katalysator gegenüber dem Beispiel 4 auf 1/3 und an Polymerisationsinhibitor auf 40% abgesenkt, ohne dass dadurch die Reaktionsgeschwindigkeit nachließ oder die Polymerisationsneigung zugenommen hätte.

### Beispiel 11:

128,2 g (1,44 mol) Dimethylaminoethanol, 371,8 g (4,32 mol) Methylacrylat, 0,5 g Phenothiazin und 1,25 g des nach Beispiel 1 hergestellten Katalysators wurden zusammen bei Normaldruck vorgelegt und bei einem Rücklaufverhältnis von 1:3 binnen 5 Stunden auf 100°C hochgeheizt. Das Destillat wurde bei einer Kopftemperatur von 65°C bei Normaldruck entnommen.
Der Sumpf hatte folgende Zusammensetzung:

| | |
|---|---|
| Methanol | 0,25 Gew.-% |
| Methylacrylat | 35,74 Gew.-% |
| Dimethylaminoethanol | 12,55 Gew.-% |
| Dimethylaminoethylacrylat | 51,00 Gew.-% |
| Höhersiedende Verunreinigungen | 0,46 Gew.-% |
| Selektivität | 99,1 % |

### Beispiel 12:

168,8 g N-Methyl-2,2-iminodiethanol und 713,3 g Methylacrylat wurden zusammen mit 0,9 g Phenothiazin und 4,5 g des Katalysators aus Beispiel 1 bei Normaldruck vorgelegt und verestert. Nach Abschluss der Reaktion hatte der Reaktorinhalt folgende Zusammensetzung:

| | |
|---|---|
| Methanol | 0,28 Gew.-% |
| Methylacrylat | 36,44 Gew.-% |
| N-Methyldiethanolamin | 0,08 Gew.-% |
| Monoester | 1,72 Gew.-% |
| N-Methyl-2,2-iminodiethyldiacrylat | 58,06 Gew.-% |
| Höhersiedende Verunreinigungen | 1,22 Gew.-% |
| Selektivität | 97,9 % |

Der Reaktoraustrag wurde zunächst bei 10 mbar/70°C von Leichtsiedern befreit und dann bei 0,3 mbar und 120°C Sumpftemperatur destilliert. Das erhaltene wasserklare Produkt wurde mit 1000 ppm Hydrochinonmonomethylether stabilisiert.

### Beispiel 13:

5 L eines nach Beispiel 8 hergestellten Reaktoraustrages wurden mit einem Feed von 350 g/h in eine kontinuierliche Destillationsapparatur eingespeist. Der erste Teil dieser kontinuierlichen Destillationsanlage bestand aus einer mit Füllkörpern gefüllten Auftriebskolonne mit einem Innendurchmesser von 30 mm und einer Trennleistung von 4 theoretischen Böden, sowie einer mit einer Edelstahlpackung ausgestatteten Abtriebskolonne mit Innendurchmesser 40 mm und sechs theoretischen Böden. Die Abnahme der Leichtsieder erfolgte über einen gemäß Temperatur und Rücklaufverhältnis geregelten Kolonnenkopf Der erhaltene Sumpf wurde kontinuierlich in einen Dünnschichtverdampfer eingeleitet, dessen Brüden auf den Fuß einer Füllkörperkolonne mit fünf theoretischen Böden (Rücklaufverhältnis 1:1) gegeben und dessen Kopf mittels Umlaufkühler auf 0°C gekühlt wurde. Am Kopf dieser Kolonne wurde das farblose Wertprodukt mit einer Reinheit von 99,97% erhalten und mit 1000 ppm Hydrochinonmonomethylether stabilisiert. Das am Kopf der ersten Kolonne erhaltene Destillat (4,12% Ethanol, 64,96% Ethylacrylat, 27,72% Dimethylaminoethanol (DMAE), 3,18% Dimethylaminoethylacrylat) konnte in die Reaktion wieder eingesetzt werden.

### Beispiel 14:

### Quaternierung des Dimethylaminoethylacrylats

858 g des im Beispiel 13 hergestellten Dimethylaminoethylacrylats wurden zusammen mit 291 g deionisiertem Wasser in einem 2-L-Autoklav der Firma Juchheim vorgelegt. Die Rührgeschwindigkeit wurde auf 275 UpM eingestellt und der Reaktorinhalt auf 50°C aufgeheizt. Bei dieser Temperatur wurde mit der Zudosierung von Methylchlorid begonnen, die nach 28 Minuten abgeschlossen war, wobei durch Kühlung über einen Thermostaten ein Ansteigen der Innentemperatur über 70°C verhindert wurde. Das aus einer Druckgasbombe entnommene Methylchlorid wurde mittels kontinuierlicher Wägung der Druckgasbombe kontrolliert. Es wurden 301,5 g Methylchorid = 99,5% der Theorie aufgenommen. Erhalten wurde eine farblose klare Flüssigkeit mit einem Wassergehalt von 20%.

### Beispiel 15:

### Polymerisation des erhaltenen kationischen Monomers

Aus dem Produkt aus Beispiel 14 wurde analog Beispiel 12 aus der EP 374 458 polymerisiert und wie im Beispiel 1 dieser EP beschrieben getestet. Die Ergebnisse waren vergleichbar mit dem von Elf Atochem unter der Bezeichnung "ADAME" vertriebenen Dimethylaminoethylacrylat.

### Beispiel 16:

236,9 g (2,66 mol) Diethylaminoethanol, 263,1 g (2,63 mol) Ethylacrylat, 0,5 g Phenothiazin und 1,25 g des nach Beispiel 1 hergestellten Katalysators wurden zusammen bei Normaldruck vorgelegt und bei einem. Rücklaufverhältnis von 1:7 binnen 5 Stunden auf eine Sumpftemperatur von 132°C aufgeheizt. Dabei ging bei einer Kopftemperatur von 77-78°C Destillat über.
Der Sumpf hatte folgende Zusammensetzung:

| | |
|---|---|
| Ethanol | 0,84 Gew.-% |
| Ethylacrylat | 15,25 Gew.-% |
| Diethylaminoethanol | 12,88 Gew.-% |
| Diethylaminoethylacrylat | 70,19 Gew.-% |
| Höhersiedende Verunreinigungen | 0,64 Gew.-% |
| Selektivität | 99,10 % |

### Beispiel 17:

236,9 g (2,66 mol) Diethylaminoethanol, 263,1 g (2,63 mol) Ethylacrylat, 0,5 g Phenothiazin und 1,25 g des nach Beispiel 3 hergestellten Katalysators wurden zusammen bei Normaldruck vorgelegt und bei einem Rücklaufverhältnis von 1:7 binnen 5 Stunden auf eine Sumpftemperatur von 132,3°C aufgeheizt. Dabei ging bei einer Kopftemperatur von 77-78°C Destillat über. Der Sumpf hatte folgende Zusammensetzung:

| | |
|---|---|
| Ethanol | 0,29 Gew.-% |
| Ethylacrylat | 15,95 Gew.-% |
| Diethylaminoethanol | 16,12 Gew.-% |
| Diethylaminoethylacrylat | 66,33 Gew.-% |
| Höhersiedende Verunreinigungen | 1,16 Gew.-% |
| Selektivität | 98,30 % |

### Beispiel 18:

236,9 g (2,66 mol) Diethylaminoethanol, 263,1 g (2,63 mol) Ethylacrylat, 0,5 g Phenothiazin und 1,25 g des nach Beispiel 4 hergestellten Katalysators wurden zusammen bei Normaldruck vorgelegt und bei einem Rücklaufverhältnis von 1:7 binnen 6 Stunden auf eine Sumpftemperatur von 138,8°C aufgeheizt. Dabei ging bei einer Kopftemperatur von 77-78°C Destillat über.
Der Sumpf hatte folgende Zusammensetzung:

| | |
|---|---|
| Ethanol | 0,73 Gew.-% |
| Ethylacrylat | 9,77 Gew.-% |
| Diethylaminoethanol | 12,11 Gew.-% |
| Diethylaminoethylacrylat | 75,11 Gew.-% |
| Höhersiedende Verunreinigungen | 2,09 Gew.-% |
| Selektivität | 97,30 % |

### Beispiel 19:

203,4 g (2,29 mol) Diethylaminoethanol, 296,6 g (2,97 mol) Ethylacrylat, 0,5 g Phenothiazin und 1,25 g des nach Beispiel 5 hergestellten Katalysators wurden zusammen bei Normaldruck vorgelegt und bei einem Rücklaufverhältnis von 1:7 binnen 6 Stunden auf eine Sumpftemperatur von 140,5°C aufgeheizt. Dabei ging bei einer Kopftemperatur von 77-78°C Destillat über.
Der Sumpf hatte folgende Zusammensetzung:

| | |
|---|---|
| Ethanol | 0,59 Gew.-% |
| Ethylacrylat | 9,52 Gew.-% |
| Diethylaminoethanol | 10,97 Gew.-% |
| Diethylaminoethylacrylat | 76,87 Gew.-% |
| Höhersiedende Verunreinigungen | 1,88 Gew.-% |
| Selektivität | 97,60 % |

### Beispiel 20:

203,4 g (2,29 mol) Diethylaminoethanol, 296,6 g (2,97 mol) Ethylacrylat, 0,5 g Phenothiazin und 1,25 g des nach Beispiel 6 hergestellten Katalysators wurden zusammen bei Normaldruck vorgelegt und bei einem Rücklaufverhältnis von 1:7 binnen 6 Stunden auf eine Sumpftemperatur von 140,1°C aufgeheizt. Dabei ging bei einer Kopftemperatur von 77-78°C Destillat über.
Der Sumpf hatte folgende Zusammensetzung:

| | |
|---|---|
| Ethanol | 0,52 Gew.-% |
| Ethylacrylat | 10,37 Gew.-% |
| Diethylaminoethanol | 14,32 Gew.-% |
| Diethylaminoethylacrylat | 72,14 Gew.-% |
| Höhersiedende Verunreinigungen | 1,88 Gew.-% |
| Selektivität | 96,70 % |

### Beispiel 21:

203,4 g (2,29 mol) Diethylaminoethanol, 296,6 g (2,97 mol) Ethylacrylat, 0,5 g Phenothiazin und 1,25 g des nach Beispiel 7 hergestellten Katalysators wurden zusammen bei Normaldruck vorgelegt und bei einem Rücklaufverhältnis von 1:7 binnen 6 Stunden auf eine Sumpftemperatur von 139,5°C aufgeheizt. Dabei ging bei einer Kopftemperatur von 77-78°C Destillat über.
Der Sumpf hatte folgende Zusammensetzung:

| | |
|---|---|
| Ethanol | 0,81 Gew.-% |
| Ethylacrylat | 9,16 Gew.-% |
| Diethylaminoethanol | 13,12 Gew.-% |
| Diethylaminoethylacrylat | 73,99 Gew.-% |
| Höhersiedende Verunreinigungen | 2,73 Gew.-% |
| Selektivität | 96,44 Gew.-% |

### Beispiel 22:

Aufarbeitung des Reaktoraustrages

Ein Reaktoraustrag mit 3,05 Gew.-% Hochsiedern wurde zunächst bei 17 mbar, einer Wärmeträgertemperatur von 105°C mit einer Zulaufgeschwindigkeit von 25 g pro Minute über einen Dünnschichtverdampfer Typ DVS2VA der Firma QVF gegeben, wobei eine Kopftemperatur von 66°C resultierte.
Das Destillat wurde in einem Kolben aufgefangen, in welchem sich 1000 ppm Hydrochinonmonomethylether (HQME) befand, und hatte folgende Zusammensetzung:

| | |
|---|---|
| Ethanol | 0,02 Gew.-% |
| Ethylacrylat | 2,20 Gew.-% |
| Dimethylaminoethanol | 2,13 Gew.-% |
| Dimethylaminoethylacrylat | 95,46 Gew.-% |
| Höhersiedende Verunreinigungen | 0,10 Gew.-% |

Dieses Destillat wurde in einer Batchdestillation über eine Kolonne mit 6 theoretischen Böden bei einem Vakuum von 100 mbar aufdestilliert. Bis zu einer Kopftemperatur von 90°C bei einem Rücklaufverhältnis von 5 wurde der Vorlauf abgenommen. Anschließend wurde die Vorlage gegen einen frischen Kolben mit 1000 ppm Hydrochinonmonomethylether ausgetauscht und bei einer Kopftemperatur von 102°C und einem Entnahmeverhältnis von 4:2 wurde das Produkt Dimethylaminoethylacrylat mit einer Reinheit von 99,99% erhalten.

## Patentansprüche

1. Verfahren zur Herstellung von Aminoalkyl(meth)acrylaten durch Umesterung aus C1-C4-Alkyl(meth)acrylaten und Aminoalkoholen, **dadurch gekennzeichnet, dass** die Umesterung in Gegenwart von mindestens einem Distannoxan der allgemeinen Formel (1) 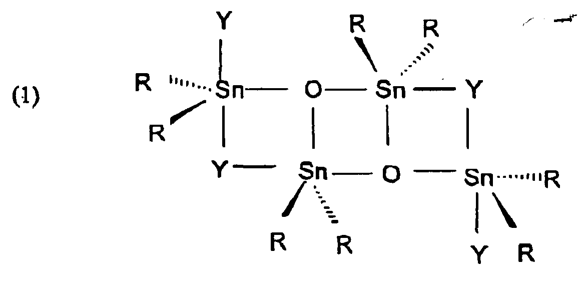 wobei
R = linearer, cyclischer oder verzweigter Alkylrest mit 1 bis 6 Kohlenstoffatomen, Phenylrest, und R identisch oder verschieden voneinander sein kann,
Y = Halogen, Pseudohalogen, OH, OAc oder OR, wobei Y identisch oder verschieden voneinander sein kann,
als Katalysator durchgeführt wird.

2. Verfahren zur Herstellung von Aminoalkyl(meth)acrylaten nach Anspruch 1, **dadurch gekennzeichnet, dass** die Umesterung in Gegenwart von mindestens einem Distannoxan der allgemeinen Formel (1) 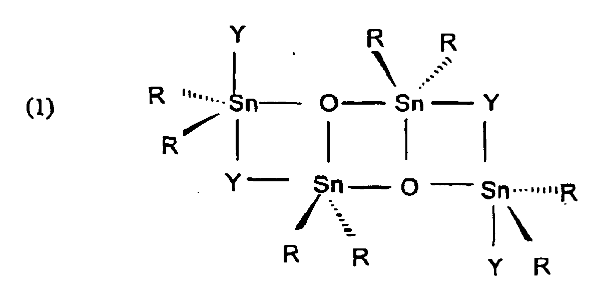 wobei
R = linearer, cyclischer oder verzweigter Alkylrest mit 1 bis 6 Kohlenstoffatomen, Phenylrest, und R identisch oder verschieden voneinander sein kann,
Y = Cl, Br, NCS, OH, OAc oder OR, wobei Y identisch oder verschieden voneinander sein kann,
als Katalysator durchgeführt wird.

3. Verfahren zur Herstellung von Anünoalkyl(meth)acrylaten nach Anspruch 1, **dadurch gekennzeichnet, dass** die Umesterung in Gegenwart von mindestens einem Distannoxan aus der Gruppe Diisopropyldibutyltetrachlordistannoxan, Düsopropyldimethyltetrachlordistannoxan, Dibutyldioctyltetrachlordistannoxan, Dimethyldioctyltetrachlordistannoxan und Dimethyldibutyltetrachlordistannoxan sowie Octabutyltetrachlordistannoxan als Katalysator durchgeführt wird.

4. Verfahren zur Herstellung von Aminoalkyl(meth)acrylaten nach Anspruch 1, **dadurch gekennzeichnet, dass** die Umesterung in Gegenwart von Octabutyltetrachlordistannoxan als Katalysator durchgeführt wird.

5. Verfahren zur Herstellung von Aminoalkyl(meth)acrylaten nach den Ansprüchen 1 bis 4, **dadurch gekennzeichnet, dass** die Einsatzmenge des Katalysators 0,1 bis 10 mmol pro Mol Aminoalkohol beträgt.

6. Verfahren zur Herstellung von Aminoakyl(meth)acrylaten den Ansprüchen 1 bis 4, **dadurch gekennzeichnet, dass** die Einsatzmenge des Katalysators 0,2 bis 1 mmol pro Mol Aminoalkohol beträgt.

7. Verfahren zur Herstellung von Aminoalkyl(meth)acrylaten nach den Ansprüchen 1 bis 6, **dadurch gekennzeichnet, dass** als Aminoalkohole Dialkylaminoalkohole eingesetzt werden.

8. Verfahren zur Herstellung von Alkylaminoalkyl(meth)acrylaten nach Anspruch 7, **dadurch gekennzeichnet, dass** als Dialkylaminoalkohole Dimethylaminoethanol, Dimethylaminopropanol, Diethylaminoethanol, Diethylaminopropanol eingesetzt werden.

9. Verfahren zur Herstellung von Aminoalkyl(meth)acrylaten nach den Ansprüchen 1 bis 8, **dadurch gekennzeichnet, dass** die Umsetzung in Gegenwart eines oder mehrer Polymerisationsinhibitoren durchgeführt wird.

10. Verfahren zur Herstellung von Aminoalkyl(meth)acrylaten nach den Ansprüchen 1 bis 9, **dadurch gekennzeichnet, dass** als Polymerisationsinhibitor Phenothiazin eingesetzt wird.

11. Verfahren zur Herstellung von Aminoalkyl(meth)acrylaten nach den Ansprüchen nach 1 bis 10, **dadurch gekennzeichnet, dass** die Umsetzung bei einem Druck im Bereich von 100 bis 1100 mbar stattfindet

12. Verfahren zur Herstellung von Aminoalkyl(meth)acrylaten nach den Ansprüchen 1 bis 11, **dadurch gekennzeichnet, dass** die hergestellten Animoalkyl(meth)acrylatc in einem kontinuierlichen Destillationsprozeß aufgearbeitet werden und die anfallende Leichtsiederfraktion in die Reaktion zurückgeführt wird.

13. Verfahren zur Herstellung von Aminoalkyl(meth)acrylaten nach Anspruch 12, **dadurch gekennzeichnet, dass** die anfallende Sumpffraktion zu 1 bis 100% in die Reaktion zurückgeführt wird.

14. Verfahren zur Herstellung von Ammoalkyl(meth)acrylaten nach Anspruch 12, **dadurch gekennzeichnet, dass** die anfallende Sumpffraktion zu 50 bis 90% in die Reaktion zurückgeführt wird

15. Verfahren nach den Ansprüchen 12 bis 14, **dadurch gekennzeichnet, dass** bei der kontinuierlichen Aufarbeitung zunächst die Hochsieder in einem Destillationsschritt abgetrennt werden, dessen Verweilzeit mit thermischer Belastung kleiner als 10 Minuten, ist.

16. Verfahren nach den Ansprüchen 12 bis 14, **dadurch gekennzeichnet, dass** bei der kontinuierlichen Aufarbeitung zunächst die Hochsieder in einem Destillationsschritt abgetrennt werden, dessen Verweilzeit mit thermischer Belastung kleiner als 1 Minute, ist.

## Claims

1. A method for the preparation of aminoalkyl (meth)-acrylates from C₁-C₄ alkyl (meth)acrylates and aminoalcohols by transesterification, **characterised in that** said transesterification is carried out in the presence of at least one distannoxane of general formula (1) as catalyst, 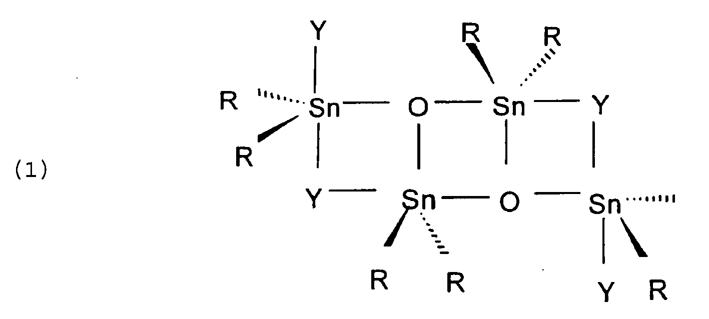 wherein
R = linear, cyclic or branched alkyl residue having from 1 to 6 carbon atoms, phenyl residue, and R can be identical or different,
Y = halogen, pseudohalogen, OH, OAc or OR, and Y can be identical or different.

2. The method for the preparation of aminoalkyl (meth)-acrylates according to claim 1, **characterised in that** said transesterification is carried out in the presence of at least one distannoxane of general formula (1) as catalyst, 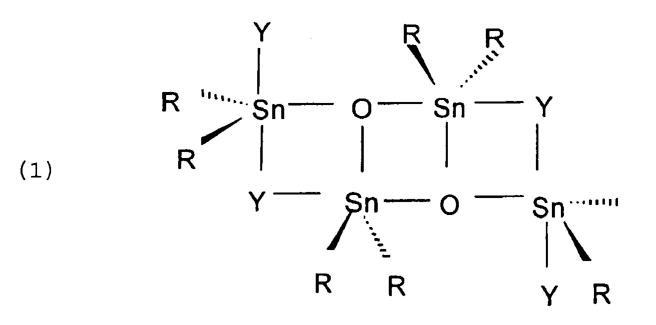 wherein
R = linear, cyclic or branched alkyl residue having from 1 to 6 carbon atoms, phenyl residue, and R can be identical or different,
Y = Cl, Br, NCS, OH, OAc or OR, and Y can be identical or different.

3. The method for the preparation of aminoalkyl (meth)-acrylates according to claim 1, **characterised in that** said transesterification is carried out in the presence of at least one distannoxane from the group of diisopropyldibutyltetrachlorodistannoxane, diisopropyldimethyltetrachlorodistannoxane, dibutyldioctyltetrachlorodistannoxane, dimethyldioctyltetrachlorodistannoxane, dimethyldibutyltetrachlorodistannoxane and octabutyltetrachlorodistannoxane as catalyst.

4. The method for the preparation of aminoalkyl (meth)-acrylates according to claim 1, **characterised in that** the transesterification is carried out in the presence of octabutyltetrachlorodistannoxane as catalyst.

5. The method for the preparation of aminoalkyl (meth)-acrylates according to claims 1 to 4, **characterised in that** the amount of catalyst used is from 0.1 to 10 mmol per mol aminoalcohol.

6. The method for the preparation of aminoalkyl (meth)-acrylates according to claims 1 to 4, **characterised in that** the amount of catalyst used is from 0.2 to 1 mmol per mol aminoalcohol.

7. The method for the preparation of aminoalkyl (meth)-acrylates according to claims 1 to 6, **characterised in that** dialkylaminoalcohols are used as aminoalcohols.

8. The method for the preparation of aminoalkyl (meth)-acrylates according to claim 7, **characterised in that** dimethylaminoethanol, dimethylaminopropanol, diethylaminoethanol, diethylaminopropanol are used as dialkylaminoalcohols.

9. The method for the preparation of aminoalkyl (meth)-acrylates according to claims 1 to 8, **characterised in that** the reaction is carried out in the presence of one or more polymerisation inhibitors.

10. The method for the preparation of aminoalkyl (meth)-acrylates according to claims 1 to 9, **characterised in that** phenothiazine is used as polymerisation inhibitor.

11. The method for the preparation of aminoalkyl (meth)-acrylates according to claims 1 to 10, **characterised in that** the reaction proceeds at a pressure ranging from 100 to 1100 mbar.

12. The method for the preparation of aminoalkyl (meth)-acrylates according to claims 1 to 11, **characterised in that** the aminoalkyl (meth)acrylates being prepared are worked up in a continuous distillation process and the low-boiling fraction obtained is fed back into the reaction.

13. The method for the preparation of aminoalkyl (meth)-acrylates according to claim 12, **characterised in that** from 1 to 100% of the bottom fraction obtained is fed back into the reaction.

14. The method for the preparation of aminoalkyl (meth)-acrylates according to claim 12, **characterised in that** from 50 to 90% of the bottom fraction obtained is fed back into the reaction.

15. The method according to claims 12 to 14, **characterised in that** in the continuous work-up, the high-boiling components are first separated off in a distillation step in which the residence time under thermal load is less than 10 minutes.

16. The method according to claims 12 to 14, **characterised in that** in the continuous work-up, the high-boiling components are first separated off in a distillation step in which the residence time under thermal load is less than one minute.

## Revendications

1. Procédé de production d'aminoalkyl(méth)acrylates par transestérification de (méth)acrylates d'alkyles en C1-C4 et d'aminoalcools,
**caractérisé en ce que**
la transestérification est réalisée en présence d'au moins un distannoxane répondant à la formule générale (1) 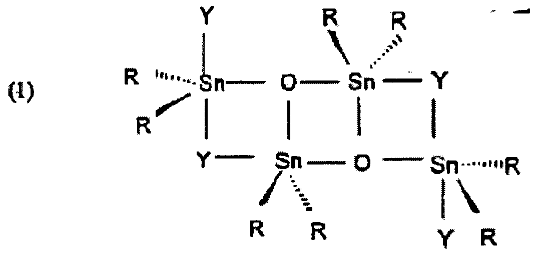 dans laquelle
R = résidu alkyle linéaire, cyclique ou ramifié ayant de 1 à 6 atomes de carbone, résidu phényle, et R peut être identique ou différent,
Y = halogène, pseudohalogène, OH, OAc ou OR, et Y peut être identique ou différent,
en tant que catalyseur.

2. Procédé de production d'aminoalkyl(méth)acrylates selon la revendication 1,
**caractérisé en ce que**
la transestérification est réalisée en présence d'au moins un distannoxane répondant à la formule générale (1) 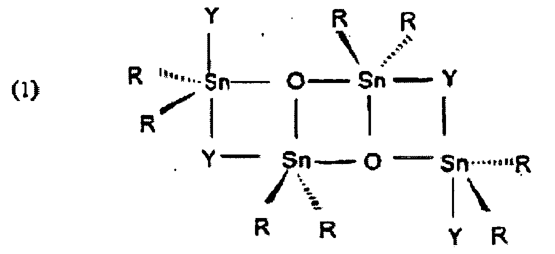 dans laquelle
R = résidu alkyle linéaire, cyclique ou ramifié ayant de 1 à 6 atomes de carbone, résidu phényle, et R peut être identique ou différent,
Y = Cl, Br, NCS, OH, OAc ou OR, et Y peut être identique ou différent, en tant que catalyseur.

3. Procédé de production d'aminoalkyl(méth)acrylates selon la revendication 1,
**caractérisé en ce que**
la transestérification est réalisée en présence d'au moins un distannoxane du groupe düsopropyldibutyltétrachrlorodistannoxane, diisopropyldiméthyltétrachlorodistannoxane,dibutyldioctyltétrachlorodistann oxane, diméthyldioctyltétrachloroditan-noxane et diméthyldibutyltétrachlorodistannoxane ainsi qu'octabutyltétrachlorodistannoxane en tant que catalyseur.

4. Procédé de production d'aminoalkyl(méth)acrylates selon la revendication 1,
**caractérisé en ce que**
la transestérification est réalisée en présence d'octabutyltétrachlorodistannoxane en tant que catalyseur.

5. Procédé de production d'aminoalkyl(méth)acrylates selon les revendications 1 à 4,
**caractérisé en ce que**
la quantité utilisée de catalyseur est de 0,1 à 10 mmol par mole d'aminoalcool.

6. Procédé de production d'aminoalkyl(méth)acrylates selon les revendications 1 à 4,
**caractérisé en ce que**
la quantité utilisée de catalyseur est de 0,2 à 1 mmol par mole d'aminoalcool.

7. Procédé de production d'aminoalkyl(méth)acrylates selon les revendications 1 à 6,
**caractérisé en ce que**
comme aminoalcools on utilise des dialkylaminoalcools.

8. Procédé de production d'aminoalkyl(méth)acrylates selon la revendication 7,
**caractérisé en ce que**
comme dialkylaminoalcools on utilise du diméthylaminoéthanol, du diméthylaminopropanol, du diéthylaminoéthanol, du diéthylaminopropanol.

9. Procédé de production d'aminoalkyl(méth)acrylates selon les revendications 1 à 8,
**caractérisé en ce que**
la conversion est réalisée en présence d'un ou de plusieurs inhibiteurs de polymérisation.

10. Procédé de production d'aminoalkyl(méth)acrylates selon les revendications 1 à 9,
**caractérisé en ce que**
comme inhibiteur de polymérisation on utilise de la phénothiazine.

11. Procédé de production d'aminoalkyl(méth)acrylates selon les revendications 1 à 10,
**caractérisé en ce que**
la conversion a lieu à une pression dans la gamme de 100 à 1 100 mbar.

12. Procédé de production d'aminoalkyl(méth)acrylates selon les revendications 1 à 11,
**caractérisé en ce que**
les aminoalkyl(méth)acrylates produits sont traités dans un procédé de distillation en continu et la fraction à bas point d'ébullition obtenue est recyclée dans la réaction.

13. Procédé de production d'aminoalkyl(méth)acrylates selon la revendication 12,
**caractérisé en ce que**
la fraction de fond obtenue est recyclée pour 1 à 100 % dans la réaction.

14. Procédé de production d'aminoalkyl(méth)acrylates selon la revendication 12,
**caractérisé en ce que**
la fraction de fond obtenue est recyclée pour 50 à 90 % dans la réaction.

15. Procédé selon les revendications 12 à 14,
**caractérisé en ce que**
pour le traitement en continu les résidus à point d'ébullition élevé sont tout d'abord séparés dans une étape de distillation dont le temps de séjour avec contrainte thermique est inférieur à 10 minutes.

16. Procédé selon les revendications 12 à 14,
**caractérisé en ce que**
pour le traitement en continu les résidus à point d'ébullition élevé sont tout d'abord séparés dans une étape de distillation dont le temps de séjour avec contrainte thermique est inférieur à 1 minute.
